# EUROPEAN PATENT APPLICATION

(11) **EP 3 875 094 A1**
(43) Date of publication of application: **08.09.2021**
(21) Application number: 20160498.0
(22) Date of filing: 02.03.2020
(51) Int. Cl.: A61K 31/519

(54) **FOLATE PREPARATIONS FOR THE TREATMENT OF OCULAR DISEASES**

(71) Applicant: Aprofol AG, 9050 Appenzell Steinegg (CH)
(72) Inventor: Ulmann, Martin, 8447 Dachsen (CH); Wiesler, Gerd, 8235 Lohn (CH); Flammer, Josef, 4102 Binningen (CH)
(74) Representative: Gernet Althaus IP AG

(57) **Abstract**

A composition is described comprising at least one folate which is useful in the treatment of ocular diseases linked to elevated retinal venous pressure.

## Description

The present invention relates to the technical field of folate compositions and the management or treatment of ocular diseases that are linked to elevated retinal venous pressure.

Diseases and degenerative conditions of the optic nerve and retina are the leading causes of visual impairment or blindness in the world. Worldwide there are approximately 300 million people suffering from different forms of visual impairment caused by eye disorders. A high share of visual impairment, around eighty percent, could either be prevented or cured.

There are various forms of retinopathies. Examples for retinopathies are diabetic (DR), hypertensive and genetic retinopathy.

Diabetic retinopathy, also known as diabetic eye disease, is a medical condition in which damage occurs to the retina due to diabetes mellitus. It is a leading cause of blindness in developed countries.

Diabetic retinopathy affects up to 80 percent of those who have had diabetes for 20 years or more. At least 90% of new cases could be reduced with efficient treatment and monitoring of the eyes. The longer a person has diabetes, the higher his or her chances of developing diabetic retinopathy.

Diabetic retinopathy often has no early warning signs. Even macular edema, which can cause rapid vision loss, may not have any warning signs for some time. In general, however, a person with macular edema is likely to have blurred vision, making it hard to do things like read or drive. In some cases, the vision will get better or worse during the day.

The first stage, also called non-proliferative diabetic retinopathy (NPDR), has no symptoms. Patients may not notice the signs and have a normal vision. The only way to detect NPDR is by fundus photography, in which micro-aneurysms (microscopic blood-filled bulges in the artery walls, capillary outpouchings) can be seen. If there is reduced vision, fluorescein angiography can show narrowing or blocked retinal blood vessels clearly (lack of blood flow or retinal ischemia), i.e there are non-perfused tissue areas. Also, intraretinal hemorrhages, lipid exudates and retinal edema, retinal microinfarctions, IRMA as well as venous beading and increased retinal venous pressure can be observed.

Macular edema, in which blood vessels leak their contents into the macular region, can occur at any stage of NPDR. Its symptoms are blurred vision and darkened or distorted images that are not the same in both eyes. Ten percent (10%) of diabetic patients will have vision loss related to macular edema. Optical Coherence Tomography can show areas of retinal thickening due to fluid accumulation from macular edema.

In the second stage, abnormal new blood vessels (neovascularisation linked to higher VEGF levels) form at the back of the eye as part of proliferative diabetic retinopathy (PDR); these can burst and bleed (vitreous hemorrhage) and blur the vision, because these new blood vessels are fragile. The first time this bleeding occurs, it may not be very severe. In most cases, it will leave just a few specks of blood, or spots floating in a person's visual field, though the spots often go away after a few hours.

Macular degeneration (MD) is yet another frequently occurring optic disorder. MD designates the loss of photoreceptors in the portion of the central retina, termed the macula, responsible for high-acuity vision. Age-related macular degeneration (AMD) is described as either "dry" or "wet." The wet, exudative, neovascular form of AMD affects about 10% of those with AMD and is characterized by abnormal blood vessels growing through the retinal pigment epithelium (RPE), resulting in hemorrhage, exudation, scarring, forming of drusens or serous retinal detachment. Ninety percent of AMD patients have the dry form characterized by atrophy of the retinal pigment epithelium and loss of macular photoreceptors.

Although some success in attenuation has been obtained with photodynamic therapy and VEGF-inhibitor there is at present no effective cure for any form of MD or AMD.

In Wang J. et al., Eye and Vision, 2019,6:21, reports on a series of cases wherein patients with non-proliferative diabetic retinopathy or hypertensive retinopathy were treated with a nonprescription multivitamin composition. The patients all had one or more MTHFR polymorphism. The primary findings were an improvement in retinal hemorrhages and micro-aneurysms as well as a reduction of exudates and macular edema. However, retinal blood flow did not seem to be significantly altered.

In Cybulska-Heinrich et al., The EMPA Journal, 2015, 6:5, a link between diabetic retinopathy (DR) and (high) retinal venous pressure was described. The results showed a marked increase in RVP in diabetes patients with DR. RVP was not increased in diabetes patients without DR. In the tested population, diabetes itself seemed to have only a minor influence on intraocular pressure.

Glaucoma is a condition resulting from several distinct systemic and eye diseases that cause vision loss by damage to the optic nerve. Elevated intraocular pressure (IOP) due to inadequate ocular drainage is the most frequent cause of glaucoma. Glaucoma often develops as the eye ages, or it can occur as the result of an eye injury, inflammation, and tumor or in advanced cases of cataract or diabetes. It can also be caused by the increase in IOP caused by treatment with steroids. Drug therapies that are proven to be effective in glaucoma reduce IOP either by decreasing aqueous humor production or by facilitating ocular draining. Such agents may be vasodilators, such as carbanhydrase inhibitors, and as such act on the sympathetic nervous system and include adrenergic antagonists. Other agents may be neutral regarding vasodilatation, such as prostaglandin analogs, or are mild vasoconstrictors, such as betablockers, or strong vasoconstrictors such as alpha-2 agonists.

There are different forms of glaucoma. Pseudoexfoliation syndrome, often abbreviated as PEX and sometimes as PES or PXS, is an aging-related systemic disease manifesting itself primarily in the eyes which is characterized by the accumulation of microscopic granular amyloid-like protein fibers. The exfoliation syndrome (XFS) is an age-related disease in which abnormal fibrillar extracellular material is produced and accumulates in many ocular tissues. Its ocular manifestations involve all of the structures of the anterior segment, as well as conjunctiva and orbital structures. Normal-tension glaucoma (NTG), also known as low tension or normal pressure glaucoma, is a form of glaucoma in which damage occurs to the optic nerve without eye pressure exceeding the normal range. In general, a "normal" pressure range is between 12-22 mm Hg.

The Flammer syndrome (FS) describes the phenotype of people with a predisposition for an altered reaction of the blood vessels to stimuli like coldness, emotional stress or high altitude. Frequent symptoms are: cold hands and/or feet, low blood pressure, prolonged sleep onset time, reduced feeling of thirst, increased sensitivity to odour, pain, vibration and certain drugs. FS subjects are often ambitious and successful but also perfectionistic and sometimes brooding. Frequent signs are: altered gene expression, prolonged blood flow cessation in nailfold capillaroscopy after cold provocation, reduced autoregulation of ocular blood flow, and reduced vasodilation after stimulation with flickering light. Retinal venous pressure is on the average higher and retinal astrocytes are more often activated. FS occurs more often in females than in males, in thin than in obese subjects, in young than in old people, in graduates than in blue collar workers, in subjects with indoor than outdoor jobs. Associated diseases are: normal tension glaucoma, occlusion of ocular vessels, retinitis pigmentosa, multiple sclerosis, tinnitus or even sudden hearing loss. In addition, small vessels disease in the brain seems to be linked to FS.

Small vessels disease (also termed microvascular disease, or microangiopathy) is an angiopathy, i.e. disease of blood vessels, affecting small blood vessels in the body. Coronary small vessel disease is a type of coronary heart disease (CHD) that affects the arterioles and capillaries of the heart. Cerebral small vessel disease refers to a group of diseases that affect the small arteries, arterioles, venules, and capillaries of the brain. Age-related and hypertension-related small vessel diseases and cerebral amyloid angiopathy are the most common forms. Cerebral small vessel disease (SVD) is an umbrella term covering a variety of abnormalities related to small blood vessels in the brain. The small vessels disease is known to contribute to vascular cognitive impairment and vascular dementia. In Smallwood et al., Neuropathology and Applied Neurobiology, 2012, 38, 337-343, cerebral small vessels disease is described as prominent cause of cognitive impairment in elderly persons, second only to Alzheimer's disease. Using an image-based scoring system the study demonstrated a significant correlation between the SVD pathology severity and cognitive impairment.

In Fang et al, BMC Ophthalmology, 2014 14, 121, the effect of the FS on retinal venous pressure is investigated. The aim of the study was to measure the retinal venous pressure (RVP) in the eyes of primary open-angle glaucoma (POAG) patients and healthy subjects with and without FS. Results showed that the RVP is higher in subjects with FS, particularly in FS subjects with glaucoma.

The currently available treatments are effective in slowing down the progression of eye disorders. They however, do not cure the eye disorders. There is an ongoing need for effective treatments for ocular or eye disorders such as macular degeneration (MD), age-related macular degeneration (AMD), diabetic retinopathy (DR), retinal and choroidal ischemia, glaucoma, cataracts, retinitis pigmentosa, choroidal neo-vascularization, retinal degeneration, and ocular surface diseases.

Intracranial pressure (ICP) is the pressure inside the skull and thus in the brain tissue and cerebrospinal fluid (CSF). ICP is usually measured in mmHg. At rest, it is normally 7-15 mmHg for a supine adult, that is, an adult lying horizontally with the face and torso facing up. The body has various mechanisms by which it keeps the ICP stable, with CSF pressures varying by about 1 mmHg in normal adults through shifts in production and absorption of CSF. Changes in ICP are attributed to volume changes in one or more of the constituents contained in the cranium. Intracranial hypertension, commonly abbreviated IH, IICP or raised ICP, is elevation of the pressure in the cranium. ICP is normally 7-15 mm Hg; at 20-25 mm Hg, the upper limit of normal, treatment to reduce ICP may be needed.

In Pillunat KR, et al., Br J Ophthalmol 2014, 98,1374-1378 the role of central retinal venous pulsation pressure (CRVPP) was evaluated in patients with intraocular pressure (IOP)-controlled open angle glaucoma patients with early, moderate and advanced disease stage and compared to a healthy control group. In more advanced cases of glaucoma, CRVPP seemed to be much higher than previously thought.

CRVPP is a dysfunction of local microcirculation leading to reduced perfusion pressure and thereby leading to an impaired local blood supply in the respective tissue and to increased transmural pressure and thereby increasing the risk for edema. CRVPP may be considered as a common marker of the discussed diseases.

In Gugleta K, Klinisches Monatsblatt Augenheilkunde, 2018, 235, 140-145, the significance of endothelin-1 in glaucoma is described. Endothelin-1 is a ubiquitous molecule that occurs in practically all tissues. Its primary physiological function is the regulation of the blood vessel diameter and hence the regulation of the blood supply in tissues. It is secreted locally and predominantly exerts its effects locally. Endothelin-1 is involved in the regulation of blood flow in the retina and the optic nerve.

In Flammer et al., The EMPA Journal, DOI 10.1186/s13167-015-0043-1, the role of endothelin on retinal venous pressure (RVP) is described. While in healthy subjects the RVP is usually equal or slightly above intraocular pressure (IOP), it is often significantly increased in patients with eye or systemic disease.

In Alrashdi S et al., The American Journal of Pathology, vol. 188, no. 3, March 2018, a linkage between endothelin-2 and its effect in retinal tissue pathology, including inflammation, glial cell dysfunction and angiogenesis is evaluated. It seems that blocking endothelin-2 mediated effects, its detrimental effects on retinopathy can be significantly reduced.

It is an object of the present invention to provide preparations for use the treatment of diseases, in particular eye diseases that are linked to elevated retinal venous pressure.

As a normal level of retinal venous pressure (RVP) is considered a pressure to be equal or a few mm Hg above intraocular pressure (IOP). The IOP might be adjusted by the ophthalmodynamometric force (ODF) to result in RVP. That is to say, if a spontaneous venous pulsation is present (ODF = 0), RVP equals IOP, if no spontaneous venous pulsation is present, the ODF necessary to detect a spontaneous venous pulsation is measured and RVP is calculated as RVP = ODF + IOP.

Retinal venous pressure may be measured as describe for instance in Mozaffarieh M. et al., Graefes Arch Clin Exp Ophthalmol, 2014, 252, 1569-1571, by ophthalmodynamometry. The ophthalmodynamometric force may be measured as described in Mustur D. et al., The EPMA Journal, 2017, 8, 339-344.

Of course, retinal venous pressure is an important parameter not only in the ocular system but may also be a sign for a general disease or metabolic syndrome. A further microcirculation-related disease is pre-eclampsia (PE). It is a disorder of pregnancy characterized among further symptoms by the onset of high blood pressure.

A preparation according to the present invention for use in the treatment of ocular diseases comprises at least one folate or a salt thereof. The ocular disease is linked to an elevated retinal venous pressure.

Folates are reduced forms of folic acid which may also employed in preparations according to the present invention.

Folic acid is the oxidized form and the parent compound of biological folate. Because of its stability folic acid is used for supplements and food fortification. Folic acid is however not metabolically active and requires reduction and one carbon substitution before it is converted to 5-methyl tetrahydrofolate by several enzymatically catalyzed steps. While the enzymatic conversion of folic acid itself may be incomplete, disrupted or reduced at several points of its pathway, the effect of its deficiency may be multiplied as the folate metabolism is linked to other metabolic cycles which means, that a malfunction in one cycle may induce malfunctions in other metabolic cycles. A further form is 5-formyl tetrahydrofolate, wherein instead of the methyl moiety a formyl moiety is present.

In a further embodiment the preparation further comprises a sulfur donor compound, preferably N-acetyl cysteine.

In another embodiment the preparation additionally comprises at least one compound of the B vitamin complex. These B vitamins are preferably selected from the group consisting of vitamin B₁, vitamin B₂, vitamin B₆, and vitamin B₁₂. Vitamin B₁₂ is particularly preferred.

In yet another embodiment the preparation further comprises arginine or an arginine ester.

In addition, the preparation may comprise a choline donor. A preferred choline donor is betaine.

The composition may also comprise the compound acetylcholine.

Additionally, the preparation may comprise glucosamine.

In a further embodiment, the composition comprises vitamin D, wherein vitamin D₃ is particularly preferred.

In a preferred embodiment, the folate salt consists of a folate and a cation as counter ion wherein the cation is selected from the group consisting of arginine, choline, acetylcholine, 1,1-dimethylbiguanidine, phenylethylbiguanidine, glucosamine and dimethylaminoethanol.

The following folates are preferred, 5-formyl-(6S)-tetrahydrofolic acid, 5-formyl-(6RS)-tetrahydrofolic acid, 10-formyl-(6R)-tetrahydrofolic acid, 5-methyl-(6S)-tetrahydrofolic acid, 5-methyl-(6RS)-tetrahydrofolic acid, (6S)-tetrahydrofolic acid, 5,10-methylene-(6R)-tetrahydrofolic acid, 5,10-methenyl-(6R)-tetrahydrofolic acid, 5,10-diformyl-(6S)-tetrahydrofolic acid, 5-methyl-10-formyl-(6S)-tetrahydrofolic acid. That means the anion of the folate salt is selected from the group consisting of the afore-mentioned folates.

The preparation may be formulated in different forms, such as a liquid preparation, a cream or in a capsule.

A liquid form of the preparation is for instance the following, the compositions according to the instant invention comprise the calcium salt, magnesium salt, sodium or zinc salt of levoleucovorin, and one or more of the compounds sodium gluconate, potassium gluconate, sodium lactate, potassium lactate, glycerophosphate disodium salt or glycerophosphate dipotassium salt. Further cations in the salt of levoleucovorin such as arginine may be used.

The composition may or may not contain additional excipients. Preferably the compositions are free of benzyl alcohol, tromethamine or monothioglycerol. Excipients such as mannitol for acceptable cake formation during the freeze-drying process, or sodium chloride and dextrose to adjust for osmolarity may be added to the compositions. The pH of the solutions is typically in the range of 6.5 to 8.5, and can be adjusted during drug product manufacturing with e.g. small amount of hydrochloric acid or sodium hydroxide. The solution may contain antioxidants to prevent oxidative degradation.

In the composition at least one additional compound, like the calcium salt, sodium salt, magnesium salt or zinc salt of leucovorin, (6R,S)-tetrahydrofolic acid, (6S)-tetrahydrofolic acid, 5,10-methylene-(6R,S)-tetrahydrofolate, 5,10-methylene-(6R)-tetrahydrofolate, 5-methyl-(6R,S)-tetrahydrofolate or 5-methyl-(6S)-tetrahydrofolate or a mixture of 2, 3 or more of said compounds can be used.

Preferred compositions according to the instant invention comprise one or more of the compounds sodium gluconate, potassium gluconate, sodium lactate, potassium lactate, glycerophosphate dipotassium salt or glycerophosphate disodium salt.

Regarding the amounts of the compounds in the compositions the following ratios are preferred. The compositions preferably contain for one mole of the calcium salts, sodium salts, magnesium salts or zinc salts of levoleucovorin, 0.8 to 6.0 moles, advantageously 1.0 to 4.0 moles, of sodium gluconate, potassium gluconate, sodium lactate or potassium lactate. In a practical embodiment the compositions preferably contain for one mole of the calcium salts, magnesium salts or zinc salts of levoleucovorin, 1.5 to 3.0 moles of sodium gluconate, or potassium gluconate. Such a composition may contain for one mole of the salt a minimum of 0.8, preferred of 1.0 moles, advantageous 1.5 moles, and a maximum of 6.0 moles, preferably of 4.0 moles, advantageous 3.0 moles, of sodium gluconate, potassium gluconate, sodium lactate or potassium lactate.

Other compositions preferably contain for one mole of the calcium salts, sodium salts, magnesium salts or zinc salts of levoleucovorin, 0.4 to 4.0 moles, preferably 0.5 to 3.0 moles, advantageously 0.7 to 2.0, of glycerophosphate disodium salt or glycerophosphate dipotassium salt. Such a composition may contain for one mole of the salt a minimum of 0.4, preferred of 0.5 moles, advantageously 0.7 moles and a maximum of 4.0 moles, preferably of 3.0 moles, advantageously of 2.0 moles of glycerophosphate disodium salt or glycerophosphate dipotassium salt.

Another exemplary composition or preparation which is suitable to be contained in a capsule is as follows, for example, the preparation contains the components calcium salt of folate, such as L-5-methyl-tetrahydrofolate or L-5-formyl-tetrahydrofolate, a sulfur donor compound such as N-acetylcysteine or its salt, a selene comprising compound such as L-selenomethionine, cholecalciferol, calcium D-panthothenate, vitamin B₁₂, such as methylcobalamin, vitamin B₆, such as pyridoxal-5'-phosphate, vitamin B₂, such as riboflavin, vitamin B₁, such as thiamine mononitrate, zeaxanthin, lutein, vitamin E, namely D-a-tocopherol, vitamin C, such as calcium ascorbate, a gluconate such as copper gluconate and zinc comprising compound, such as zinc oxide or zinc acetate.

For example, the preparation contains the components calcium salt of L-5-methyl-tetrahydrofolate in an amount of 0.2mg to 2.7mg, N-acetylcysteine or its salt in an amount of 40mg to 540mg, L-selenomethionine in an amount of 0.005mg to 0.06mg, cholecalciferol in an amount from 0.009mg to 0.1 mg, calcium D-panthothenate in an amount from 1 mg to 15mg, methylcobalamin in an amount from 0.003mg to 0.1.5mg, pyridoxal-5'-phosphate in an amount of 1mg to 9mg, riboflavin in an amount of 2mg to 30mg, thiamine mononitrate in an amount of 0.2mg to 4.5mg, zeaxanthin in an amount from 1mg to 3mg, lutein in an amount from 4mg to 15mg, D-α-tocopherol in an amount from 1mg to 16mg, calcium ascorbate in an amount from 20mg to 100mg, copper gluconate in an amount from 0.1 to 2mg and zinc oxide in an amount from 7mg to 80mg.

The component N-acetylcysteine can be used both in the form of the free acid and in the form of one of its salts, for example as a sodium or calcium salt. N-acetylcysteine may also be present in the form of N-acetylcysteine-amide. A further sulfur donor compound is lipoic acid.

The composition further preferably contains one or more auxiliary substances selected from the group consisting of refined soybean oil, type NGM, soybean oil, partially hydrogenated 31°-37°C, soybean oil, partially hydrogenated 36°C-42°C, soy lecithin and beeswax. The soya oils provide protection of the active components against light and moisture.

In another preferred version, the composition contains one or more auxiliaries selected from the group consisting of refined soybean oil, type NGM, soybean oil, partially hydrogenated 31°-37°C, soybean oil, partially hydrogenated 36°C-42°C, soy lecithin, beeswax, triglycerides of medium or longer chain length (medium chain length: C₆ to C₁₂, longer chain length: C₁₃ to C₂₄), phosphoglycerides with at least one organic phosphoric acid ester, and ester compounds from alcohols with a chain length of C₂₀ to C₄₀ and fatty acids with medium or long chain length.

In another version, the composition is contained in a soft gelatine capsule. The soft gelatine capsule preferably contains gelatine, glycerol 98%, glycerol 86%, sorbitol 70%, titanium dioxide, sodium copper chlorophylline and water.

An exemplary formulation for a cream comprises thoroughly mixed phases. Phase A comprises an oil. Phase B comprises glycerol and an emulsifier. The phase C comprises an aqueous folate composition comprising a physiologically effective amount of a folate salt, and optionally at least one compound selected from the group consisting of sodium gluconate, potassium gluconate, glycerophosphate disodium salt and glycerophosphate dipotassium salt. It may optionally comprise further compounds such as a pharmaceutically acceptable buffer, e.g. tris(hydroxymethyl)-aminomethan (TRIS), and a pharmaceutically acceptable antioxidant, e.g. glutathione. Optional phase D comprises one or more matting agents. Titanium dioxide may be used as a white dye. Alternatively, silica dioxide can be used.

A folate preparation according to the present invention preferably comprises in phase C 0.1 mg to 1000 mg at least one folate salt selected of the group consisting of calcium, magnesium, sodium, zinc, arginine, choline, acetylcholine, 1,1-dimethylbiguanidine, phenylethylbiguanidine, and dimethylaminoethanol salt of the folate per milliliter of a polar solvent. Polar solvents are for instance water, methanol, ethanol, n-propanol, isopropanol, glycerine, dimethylsulfoxide. Mixtures of such polar solvents may also be used. Arginine, choline, acetylcholine, 1,1-dimethylbiguanidine, phenylethylbiguanidine, glucosamine and dimethylaminoethanol salt of the folate are preferred.

In another embodiment the phase C comprises for one mole of the folate salt 0.8 to 10 molar sodium gluconate or potassium gluconate. If phase C comprises glycerophosphate disodium salt or glycerophosphate dipotassium salt, these salts are preferably present in a concentration of 0.4 to 5 molar.

Preferably, preparations according to the present invention comprise in phase A an oil consisting of a medium-chain triglycerol. Fatty acid of such medium-chain triglycerols have a chain-length in the range of C₆ to C₁₂. Most preferred is the oil of caprylic/capric acid triglycerol. Further, it may optionally comprise a dicarbonic acid alcohol. The dicarbonic acid has a chain length in the range of C₂ to C₁₀ and the alcohol is selected of the group consisting of methyl, ethyl, isopropyl, propyl, butyl, pentyl alcohol.

Emulsifiers used in phase B of preparations preferably have HLB value equal or greater than 5. The hydrophilic-lipophilic balance of a surfactant is a measure of the degree to which it is hydrophilic or lipophilic, determined by calculating values for the different regions of the molecule, as described by Griffin in 1954. Griffin's method for non-ionic surfactants as described in 1954 works as follows: HLB = 20 x Mh/M, wherein Mh is the molecular weight of the hydrophilic portion of the compound and M is the total molecular weight of the compound. An HLB value of 0 corresponds to a completely lipophilic/hydrophobic molecule, and a value of 20 corresponds to a completely hydrophilic/lipophobic molecule. An emulsifier having HLB value in the range of 8 to 16 is suitable for stabilizing oil in water (o/w) emulsions.

A preferred emulsifier is a sucrose ester, wherein the fatty acids have chain length in the range of C₁₄ to C₂₀. Such sucrose esters must have a HLB value equal or greater than 5. Most preferred is the emulator sucrose stearate.

### Examples

In a preliminary study a patient was treated with Oculfolin Forte®, a folate preparation as described above, wherein the preparation is comprised in a capsule. The treatment was performed for ten days. The retinal venous pressure of the patient was measured as indicated before the treatment cycle and thereafter in both eyes. Before the treatment the RVP was measures as 56 mmHg (right eye) and 59 mmHg (left eye). The intraocular pressure (IOP) was determined to be 18 mmHg (both eyes). After the treatment the RVP was determined at 32 mmHg (right eye) and 16 mmHg (left eye). The IOP was measured as 16 mmHg (both eyes). The results show a massive decrease of the RVP.

Further measurement with glaucoma patients gave the following results:

**Table 1**

| | | | Retinal Venous Pressure | |
|---|---|---|---|---|
| | | | Right eye | Left eye |
| Patient | Date | HCy | mm Hg | mm Hg |
| 1 | 21.11.19 | 12.89 | | |
| | 16.01.20 | | 25.5 | 74,1 |
| | 13.02.20 | | 16.5 | 58 |
| | | | | |
| 2 | 4.11.19 | 12.63 | 35.90 | 39.9 |
| | 24.01.20 | | 21.90 | 27 |
| | 31.01.20 | | 14.00 | 18 |
| | | | | |
| 3 | 26.11.19 | 18.63 | 28.8 | 71 |
| | 6.02.20 | | 18 | 40 |
| | | | | |
| 4 | 21.11.19 | 15.41 | | |
| | 9.01.20 | | 32.90 | 34.1 |
| | 23.01.20 | | 15.40 | 17.3 |

Patients had supplementation with Ocufolin Forte® (one capsule per day).

Further, table 2 shows the results from a study with diabetes patients treated with Ocufolin Forte. The levels of homocysteine (HCy) and endothelin-1 were determined before and after the treatment. In all cases except one a massive decrease of up to 75% in the HCy and endothelin levels could be measured.

**Table 2**

| Patient No. | Treatment | Date | HCy | Endothelin |
|---|---|---|---|---|
| | | | µmol/ l | pg/ ml |
| 1 | before | 21.02.2019 | 13.9 | 2.46 |
| | after | 06.06.2019 | 5.9 | 1.39 |
| 2 | before | 18.03.2019 | 38.7 | 1.84 |
| | after | 12.06.2019 | 8.70 | 1.39 |
| 3 | before | 07.03.2019 | 21.2 | 2.59 |
| | after | 06.06.2019 | 15.1 | 1.89 |
| 4 | before | 05.04.2019 | 10.9 | 1.70 |
| | after | 12.07.2019 | 5.8 | 1.28 |
| 5 | before | 17.04.2019 | 6.10 | 1.53 |
| | after | 24.07.2019 | 6.5 | 0.79 |
| 6 | before | 22.05.2019 | 11.7 | 2.74 |
| | after | 14.08.2019 | 7.8 | 1.66 |

## Claims

1. A preparation comprising at least one folate for use in the treatment of ocular diseases wherein the disease is linked to elevated retinal venous pressure.

2. A preparation according to claim 1, **characterized in that** the preparation further comprises a sulfur donor compound, preferably N-acetylcysteine.

3. A preparation according to claim 1 or two, **characterized in that** the preparation further comprises at least one vitamin of the B complex, preferably selected from the group consisting of vitamin B₁, vitamin B₂, vitamin B₆, and vitamin B₁₂.

4. A preparation according to any of claims 1 to 3, **characterized in that** the preparation further comprises arginine or arginine esters.

5. A preparation according to any of claims 1 to 4, **characterized in that** the preparation further comprises a choline donor, preferably betaine.

6. A preparation according to any of claims 1 to 5, **characterized in that** the preparation further comprises acetylcholine.

7. A preparation according to any of claims 1 to 6, **characterized in that** the preparation further comprises glucosamine.

8. A preparation according to any of claims 1 to 7, **characterized in that** the preparation further comprises vitamin D, preferably vitamin D₃.

9. A preparation according to any one of claims 1 to 8 **characterized in that** the cation of the folate salt is selected from the group consisting of arginine, choline, acetylcholine, 1,1-dimethylbiguanidine, phenylethylbiguanidine, glucosamine and dimethylaminoethanol.

10. A preparation according to any one of claims 1 to 10, **characterized in that** the anion of the folate salt is selected from the group consisting of 5-formyl-(6S)-tetrahydrofolic acid, 5-formyl-(6RS)-tetrahydrofolic acid, 10-formyl-(6R)-tetrahydrofolic acid, 5-methyl-(6S)-tetrahydrofolic acid, 5-methyl-(6RS)-tetrahydrofolic acid, (6S)-tetrahydrofolic acid, 5,10-methylene-(6R)-tetrahydrofolic acid, 5,10-methenyl-(6R)-tetrahydrofolic acid, 5,10-diformyl-(6S)-tetrahydrofolic acid, 5-methyl-10-formyl-(6S)-tetrahydrofolic acid.
